# EUROPEAN PATENT APPLICATION

(11) **EP 4 289 920 A1**
(43) Date of publication of application: **13.12.2023**
(21) Application number: 22305831.4
(22) Date of filing: 08.06.2022
(51) Int. Cl.: C10M 145/22, A61K 47/44, C08F 2/24, C08F 2/48, C08L 91/02

(54) **METHOD FOR MANUFACTURING ACRYLATED EPOXIDIZED PLANT OIL PARTICLES**

(71) Applicant: Paris Sciences et Lettres, 75006 Paris (FR); SORBONNE UNIVERSITE, 75006 Paris (FR); Centre National de la Recherche Scientifique (CNRS), 75016 Paris (FR)
(72) Inventor: FATTACCIOLI, Jacques, 75005 PARIS (FR); HU, Chaohe, 75005 PARIS (FR)
(74) Representative: Casalonga

(57) **Abstract**

The present invention relates to a method for manufacturing acrylated epoxidised plant oil particles comprising the following steps:
a) mixing acrylated epoxidised plant oil with at least one solvent and in the presence of at least one photoinitiator;
b) optionally mixing the solution after step a) with a solution S1;
c) mixing the solution obtained after step a) or after optional step b) with an aqueous solution comprising at least one surfactant in order to obtain droplets;
d) exposing said droplets to a UV light;
e) retrieving the acrylated epoxidised plant oil particles obtained after step d).

## Description

### Technical field

The present invention relates to a method for manufacturing acrylated epoxidised plant oil particles. The invention also relates to acrylated epoxidised plant oil particles obtained by the method according to the invention. The present invention also relates to the use of acrylated epoxidised plant oil particles obtained by the method according to the invention for charging fluorescent or for releasing biological molecules or as optical lens.

### Prior art

Seeking materials based on fossil fuels, in particular polymer materials, has greatly boosted the development of human society. Nevertheless, with continuous utilisation of resources and the generalised use of petroleum-based polymers, numerous environmental problems have gradually arisen over the last decades, such as the energy crisis or environmental pollution.

In this context, the eco-material concept was proposed in the early 1990s. Said eco-materials denote materials that are environmentally friendly while retaining the properties and performances thereof.

At present, more than 7% of petroleum, gas and derivatives thereof are converted into polymer materials, most of which are not recyclable and are nondegradable, which can cause serious environmental damage.

Consequently, there is an urgent need to seek an alternative raw material from renewable resources to improve the environment and reduce overdependence on petroleum. Fortunately, an abundance of renewable resources can be found in nature, which can be used to design and produce a biosourced polymer material, such as for example cellulose, chitosan, alginate, lignin, and plant oils.

Plant oils and the derivatives thereof are the most promising candidates for the crude source of biosourced materials, which can offer numerous advantages of easy access, low cost, a wide range of sources, renewal and degradability.

They are primarily composed of unsaturated triglycerides and the existence of unsaturated bonds in the molecule makes reaction modification and functionalisation possible and easy.

In recent years, a growing interest in research can be observed in developing strategies for preparing and applying plant oil-based materials. Thus, various studies have been conducted on this topic with extensive literature.

However, there is still a need to develop novel methods for manufacturing plant oil-based materials.

Thus, the aim of the present invention is that of developing a novel method for manufacturing acrylated epoxidised plant oil particles.

### Description of the invention

Therefore, the invention relates to a method for manufacturing acrylated epoxidised plant oil particles comprising the following steps:
a) mixing acrylated epoxidised plant oil with at least one solvent and in the presence of at least one photoinitiator;
b) optionally mixing the solution after step a) with a solution S1;
c) mixing the solution obtained after step a) or after optional step b) with an aqueous solution comprising at least one surfactant in order to obtain droplets;
d) exposing said droplets to a UV light;
e) retrieving the acrylated epoxidised plant oil particles obtained after step d).

The invention also relates to acrylated epoxidised plant oil particles obtained by the method according to the invention.

The invention further relates to the use of acrylated epoxidised plant oil particles for charging fluorescent molecules or for releasing biological molecules, such as ibuprofen, or as optical lens.

Thanks to the method according to the invention, acrylated epoxidised plant oil particles can be obtained. Said particles can particularly be degradable in aqueous phase in the presence of enzymes or in an alcoholic and alkaline solution.

It is specified that the expression "from... to..." used in the present description of the invention should be understood as including each of the bounds mentioned.

The expression "at least one" is equivalent to "one or more".

Further advantages and features of the invention will become clearer on examination of the detailed description and the accompanying drawings in which:
[Fig 1A] is a schematic representation of a microfluidic device employed according to one embodiment of the method according to the invention;
[Fig 1B] is another schematic representation of a microfluidic device employed according to one embodiment of the method according to the invention and of a UV light;
[Fig 2A] is another schematic representation of a microfluidic device employed according to one embodiment of the method according to the invention and of a UV light;
[Fig 2B] is an image of a particle obtained thanks to one embodiment of the method of the invention, observed with a scanning electron microscope;
[Fig 3A] is another schematic representation of a microfluidic device employed according to one embodiment of the method according to the invention and of a UV light;
[Fig 3B] is an image of a particle obtained thanks to one embodiment of the method of the invention, observed with a scanning electron microscope;
[Fig 4] is another schematic representation of two microfluidic devices connected in series employed according to one embodiment of the method according to the invention and of a UV light;
[Fig 5] is a graph representing the evolution of the degradation rate of the particles obtained by the method according to the invention according to one embodiment, as a function of time, in different solutions;
[Fig 6] is a graph representing the evolution of the concentration of ibuprofen loaded on particles obtained by the method according to the invention according to one embodiment, as a function of time, in two different environments.

As described above, according to step a), acrylated epoxidised plant oil is mixed with at least one solvent and in the presence of at least one photoinitiator.

Preferably, the acrylated epoxidised plant oil is chosen from acrylated epoxidized linseed oil, acrylated castor oil, acrylated epoxidised soybean oil and their mixtures, preferably the acrylated epoxidised plant oil is acrylated epoxidised soybean oil.

Acrylated epoxidised soybean oil (AESO) is one of the most typical plant oil derivatives. It is obtained from successive epoxidation and esterification of soybean oil, wherein the carbon-carbon double bonds are open and modified by the acrylate and hydroxyl group.

Thus, according to a preferred embodiment, the method according to the invention is used to manufacture acrylated epoxidised soybean oil particles.

Obtaining acrylated epoxidised soybean oil in particle form represents real technological progress. Indeed, acrylated epoxidised soybean oil has a very high viscosity ranging from 18000 to 32000 mPa.s. Due to this very high viscosity, research conducted in this field does not describe AESO in particle form for processability reasons.

According to a preferred embodiment, the acrylated epoxidised plant oil particles are present at a content ranging from 40 to 90% by weight, preferably from 50 to 90% by weight, more preferably from 60 to 90% by weight, even more preferably from 65 to 85% by weight with respect to the total weight of the solution obtained after step a).

According to a preferred embodiment, the solvent is chosen from alcohols, esters and their mixtures, preferably from C₆ to C₁₂ monoalcohols, C₁₂ to C₃₆ esters and their mixtures, more preferably from C₆ to C₁₂ monoalcohols, C₁₂ to C₂₄ esters and their mixtures, even more preferably from 1-octanol, methyl oleate and their mixture, even better the solvent is 1-octanol.

According to one embodiment, the photoinitiator can be any photoinitiator fitted for acrylate monomer known by the skilled person.

Advantageously, the photoinitiator is chosen from ketones, such as 2-hydroxy-2-methyl-1-phenyl-propan-1-one, 1-hydroxy-cyclohexyl-phenyl-ketone, 2,2-dimethoxy-1,2-diphenylethan-1-one, 2-hydroxy-4'-(2-hydroxyethoxy)-2-methylpropiophenone, etc., phosphine oxides, such as phenylbis(2,4,6-trimethylbenzoyl)phosphine oxide, and their mixtures, more preferably from 2-hydroxy-4'-(2-hydroxyethoxy)-2-methylpropiophenone, phenylbis(2,4,6-trimethylbenzoyl)phosphine oxide and their mixture, even more preferably the photoinitiator is phenylbis(2,4,6-trimethylbenzoyl)phosphine oxide.

Advantageously, the photoinitiator is present at a content ranging from 0.05 to 5% by weight, preferably from 0.05 to 3% by weight, more preferably from 0.05 to 1% by weight, even more preferably from 0.1 to 0.5% by weight with respect to the total weight of the solution obtained after step a).

According to one embodiment, acrylated epoxidised plant oil may be further mixed with at least one surfactant. Preferably, said surfactant may be a non-ionic surfactant. Advantageously, said surfactant may be sorbitane oleate (Span 80).

As described above, according to optional step b), the solution obtained after step b) may be mixed with a solution S1.

The solution S1 may be any type of solution, for example a water-miscible solution or a water-immiscible solution and/or an oil-miscible solution or an oil-immiscible solution.

According to one embodiment, the solution S1 can be a water-immiscible solution and an oil-immiscible solution.

According to another embodiment, the solution S1 can be a water-miscible solution and an oil-immiscible solution.

Advantageously, the solution S1 is chosen from a solution comprising water and a solution comprising a silicone oil.

According to one embodiment, the solution S1 comprises a silicone oil, for example polydimethylsiloxane, dimethicone or dimethylpolysiloxane.

More preferably, the solution S1 consists of a silicone oil such as polydimethylsiloxane. Suitable silicone oils such as polydimethylsiloxane having a viscosity ranging from 50 to 375 cSt can be cited. Silicone oil such as the silicone oil (polydimethylsiloxane) (viscosity 350 cSt at 25°C, CAS n° 63148-62-9) sold by the company Sigma-Aldrich may be cited.

According to another embodiment, the solution S1 comprises water.

According to one embodiment, the solution S1 may be an aqueous solution comprising a viscosity agent, such as alginate sodium.

As described above, according to step c), the solution obtained after step a) or after optional step b) is mixed with an aqueous solution comprising at least one surfactant in order to obtain droplets.

The surfactant can be chosen from anionic surfactants, non-ionic surfactants, and their mixtures.

Advantageously, the surfactant is chosen from poloxamer 188 (polyoxyethylene-polyoxypropylene block copolymer), sodium dodecyl sulfate, poloxamer 407 and their mixtures.

More preferably, the surfactant is chosen from non-ionic surfactants, even more preferably from poloxamer 188.

The surfactant can be present at a content ranging from 1 to 40% by weight, preferably from 5 to 30% by weight, more preferably from 10 to 20% by weight, with respect to the total weight of said aqueous solution.

Preferably, the aqueous solution comprising at least one surfactant further comprises at least one viscosity agent.

The viscosity agent can be chosen from sodium alginate.

The viscosity agent, when present, can be present at a content ranging from 0.1 to 5% by weight, preferably from 0.5 to 3% by weight, more preferably from 0.5 to 2% by weight with respect to the total weight of said aqueous solution.

The aqueous solution may further comprise polyethylene glycol sorbitan monolaurate (Tween 20).

Advantageously, both solutions can be mixed in a vortex mixer.

During step c), an emulsification can be carried out.

As described above, according to step d), the droplets are exposed to a UV light. Thus, with this step of exposure to UV light, polymerisation is carried out.

Preferably, the UV light has a wavelength ranging from 315 to 380 nm.

Advantageously, step d) is carried out for a period ranging from 0.1 second to 30 minutes, preferably from 0.5 second to 20 minutes, more preferably from 0.5 second to 10 minutes.

According to a specific embodiment, a step of isolating and rinsing the droplets is carried out between step c) and step d).

This rinsing of the droplets makes it possible to remove the surfactant and any viscosity agent when present.

The droplets can be rinsed with a solution comprising polyethylene glycol sorbitan monolaurate (Tween 20). Said solution may also comprise a phosphate buffer.

According to another specific embodiment, optional step b), and steps c) and d) are carried out in a microfluidic device comprising a glass substrate, a polydimethylsiloxane layer and a polydimethylsiloxane chip, and wherein the polydimethylsiloxane layer is located on the glass substrate and the polydimethylsiloxane chip is located on the polydimethylsiloxane layer.

The polydimethylsiloxane chip may conventionally comprise a channel system. In these channels, the solution obtained from mixing the acrylated epoxidised plant oil with at least one solvent and in the presence of at least one photoinitiator, as described above, the optional solution S1, and the aqueous solution comprising at least one surfactant, as described above, can circulate.

Advantageously, the solution obtained from mixing the acrylated epoxidised plant oil with at least one solvent and in the presence of at least one photoinitiator is added at a first end of a first channel, the aqueous solution comprising at least one surfactant is added at a first end of a second channel and the optional solution S1 may be added at a first end of a third channel.

In this specific embodiment, preferably, the added aqueous solution does not comprise any viscosity agent.

Then, the aqueous solution can be mixed with the solution obtained from mixing the acrylated epoxidised plant oil with at least one solvent and in the presence of at least one photoinitiator thanks to a junction of the first and second channels.

Or, the solution obtained from mixing the acrylated epoxidised plant oil with at least one solvent and in the presence of at least one photoinitiator can be mixed with the optional solution S1, thanks to a junction of the second and third channels. Then, the aqueous solution can be mixed with said obtained mixture thanks to a junction of channels.

Thus, the polydimethylsiloxane chip can incorporate at least one transverse junction for focusing the flow.

Droplets can thus be formed.

The microfluidic device may be connected to a microfluidic flow controller, well known from those skilled in the art, to adjust the fluid pressure circulating in the different channels. An example of such controller is sold under the trade name MFCS-EZ by the company Fluigent.

As those skilled in the art knows it, the pressure values for each channel can be set through a software, in connection with the controller.

Thanks to said controller, air can be pumped at a given and desired pressure through some tubes connecting some reservoirs containing different solutions (for example the aqueous solution, the optional solution S1 and the solution obtained from mixing the acrylated epoxidised plant oil with at least one solvent and in the presence of at least one photoinitiator) and the software. Then said solutions contained in said reservoirs can be pressed into the ends of the channels of the polydimethylsiloxane chip through some tubes connecting the reservoirs and the ends of the channels.

The pressures may range from 80 to 290 mbar.

Then, the droplets can follow a coiled fourth channel. This zone can be illuminated using a UV light. In other words, the droplets are exposed to a UV light.

The exposure to the UV light can be carried out for a period ranging from 0.1 second to 1 minute, preferably from 0.5 second to 30 seconds, more preferably 0.5 second to 10 seconds, even more preferably from 0.5 second to 5 seconds.

The UV light exposure time can be controlled for example by adjusting the extent of the UV light area by moving said UV light further away or closer.

At the outlet of the fourth channel, the acrylated epoxidised plant oil particles can be retrieved.

Advantageously, the method comprises a step f) of dispersing said acrylated epoxidised plant oil particles obtained after step e) in a solution comprising at least one photoinitiator, followed by exposure to a UV light.

According to a specific embodiment, the acrylated epoxidised plant oil particles obtained after step e) can be dispersed in a solution comprising acetone and at least one photoinitiator.

Then, said particles can be exposed to a UV light.

Preferably, the UV light has a wavelength ranging from 315 to 380 nm.

Advantageously, the photoinitiator is chosen from ketones, such as 2-hydroxy-2-methyl-1-phenyl-propan-1-one, 1-hydroxy-cyclohexyl-phenyl-ketone, 2,2-dimethoxy-1,2-diphenylethan-1-one, 2-hydroxy-4'-(2-hydroxyethoxy)-2-methylpropiophenone, etc., phosphine oxides such as phenylbis(2,4,6-trimethylbenzoyl)phosphine oxide, and their mixtures, more preferably from 2-hydroxy-4'-(2-hydroxyethoxy)-2-methylpropiophenone, phenylbis(2,4,6-trimethylbenzoyl)phosphine oxide and their mixture, even better the photoinitiator is phenylbis(2,4,6-trimethylbenzoyl)phosphine oxide.

According to a specific embodiment, the exposure to UV light during step f) is carried out for a period ranging from 30 seconds to 30 minutes, preferably from 1 minute to 10 minutes, more preferably from 1 minute to 5 minutes.

A first embodiment of the method according to the invention can be described hereinafter.

According to step a), acrylated epoxidised soybean oil can be mixed with at least one solvent, such as for example 1-octanol, and in the presence of at least one photoinitiator, for example phenylbis(2,4,6-trimethylbenzoyl)phosphine oxide (Irgacure^{®} 819). The acrylated epoxidised soybean oil content can range from 40 to 90% by weight with respect to the total weight of the solution obtained after step a), for example 85% by weight. The photoinitiator content can range from 0.05 to 5% by weight with respect to the total weight of the solution obtained after step a), for example 0.4% by weight.

Then, according to step c), the solution obtained after step a) is mixed with an aqueous solution comprising at least one surfactant in order to obtain droplets.

The surfactant can be a non-ionic surfactant such as for example poloxamer 188 (Pluronic^{®} F68). The aqueous solution can further comprise at least one viscosity agent, such as sodium alginate. The surfactant can be present at a content ranging from 1 to 40% by weight with respect to the total weight of said aqueous solution, for example 15% by weight. The viscosity agent can be present at a content ranging from 0.1 to 5% by weight with respect to the total weight of said aqueous solution, for example 2% by weight.

Both solutions can be mixed in a vortex mixer. The droplets obtained can then be retrieved, then, a step of isolating and rinsing the droplets can be carried out.

This rinsing of the droplets makes it possible to remove the surfactant and any viscosity agent.

According to step d), the droplets are exposed to a UV light. The UV light can have a wavelength ranging from 315 to 380 nm.

The exposure to the UV light can be carried out for a period ranging from 0.1 second to 30 minutes, more preferably from 1 to 10 minutes, for example for 8 minutes.

Acrylated epoxidised soybean oil particles can thus be obtained with the method according to the invention according to a first embodiment.

The particles thus obtained can be of spherical shape and have a smooth appearance.

A second embodiment according to the invention can be described hereinafter.

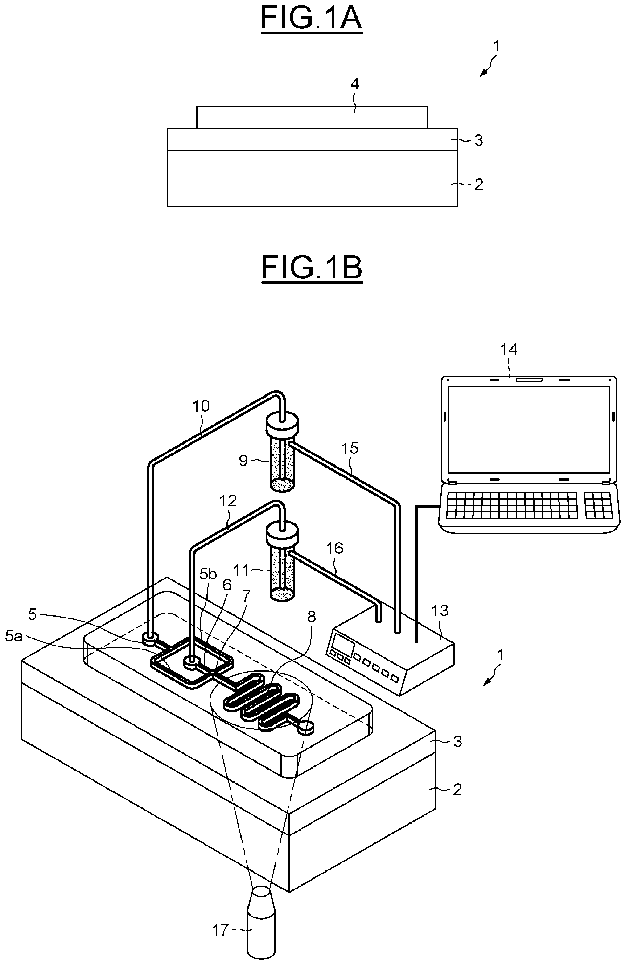

In this embodiment, a microfluidic device is used, as represented in Figures 1A and 1B.

As can be seen in Figure 1A, the device 1 may comprise a glass substrate 2, a polydimethylsiloxane layer 3 and a polydimethylsiloxane chip 4, wherein the polydimethylsiloxane layer 3 is located on the glass substrate 2 and the polydimethylsiloxane chip 4 is located on the polydimethylsiloxane layer 3. A channel system (5, 5a, 5b, 6, 7, 8) can be found inside the polydimethylsiloxane chip 4, as can be seen in Figure 1B.

According to step a), acrylated epoxidised soybean oil can be mixed with at least one solvent, such as for example 1-octanol, and in the presence of at least one photoinitiator, for example phenylbis(2,4,6-trimethylbenzoyl)phosphine oxide (Irgacure^{®} 819).

An aqueous solution comprising at least one surfactant can be prepared. The surfactant can be a non-ionic surfactant, such as for example poloxamer 188 (Pluronic^{®} F68).

The aqueous solution can then be poured into a reservoir 9 and said reservoir 9 can be connected to a first end of a channel 5 through a tube 10. The solution obtained after step a) can be poured into a reservoir 11 and said reservoir 11 can be connected to a first end of a channel 6 through a tube 12.

The reservoir 9 can be connected to a controller 13, such as for example the one sold under the trade name MFCS-EZ by the company Fluigent, and the controller 13 can be connected to a computer 14, through a tube 15. The reservoir 11 can be connected to the controller 13 through a tube 16.

Thanks to the controller 13, air is pumped at a given and desired pressure through tubes 15 and 16, respectively.

The aqueous solution comprising at least one surfactant, contained in the reservoir 9, can be added at a first end of a channel 5 through the tube 10 and the solution obtained after step a), contained in the reservoir 11, can be added at a first end of a channel 6 through the tube 12, as represented in Figure 1B. The channel 5 can be divided into two sub-channels, a first sub-channel 5a and a second sub-channel 5b.

Then, both solutions can be contacted thanks to a junction 7 of the first sub-channel 5a, the second sub-channel 5b and the second channel 6. Thus, the polydimethylsiloxane chip 4 can incorporate a transverse junction 7 for focusing the flow.

Droplets can thus be formed.

Then, the droplets can follow a coiled third channel 8, as can be seen in Figure 1B. This zone can be illuminated using a UV light 17. In other words, the droplets can be exposed to a UV light 17. Said UV light 17 can be placed outside the microfluidic device 1 and can be positioned so as to illuminate the coiled third channel 8.

The UV light can have a wavelength ranging from 315 to 380 nm. The exposure to the UV light can be carried out for a period ranging from 0.1 second to 30 minutes, more preferably from 0.5 to 5 seconds, for example for 3 seconds.

At the outlet of the third channel 8 in said chip 4, the acrylated epoxidised soybean oil particles can be retrieved.

Then, said acrylated epoxidised soybean oil particles can be dispersed in a solution comprising at least one photoinitiator, such as for example phenylbis(2,4,6-trimethylbenzoyl)phosphine oxide (Irgacure^{®} 819).

Said solution can further comprise acetone.

Said particles can then be exposed to a UV light. The UV light can have a wavelength ranging from 315 to 380 nm.

The exposure to the UV light can be carried out for a period ranging from 30 seconds to 30 minutes, for example for 2 minutes.

Acrylated epoxidised soybean oil particles can thus be obtained by the method according to the invention according to a second embodiment.

The particles thus obtained can be of different sizes depending on what is intended. Indeed, the configuration of the first and second channels can be adapted according to the pressure applied in the different channels to vary the size of said particles.

They can furthermore have a very narrow size distribution.

A third embodiment according to the invention can be described hereinafter.

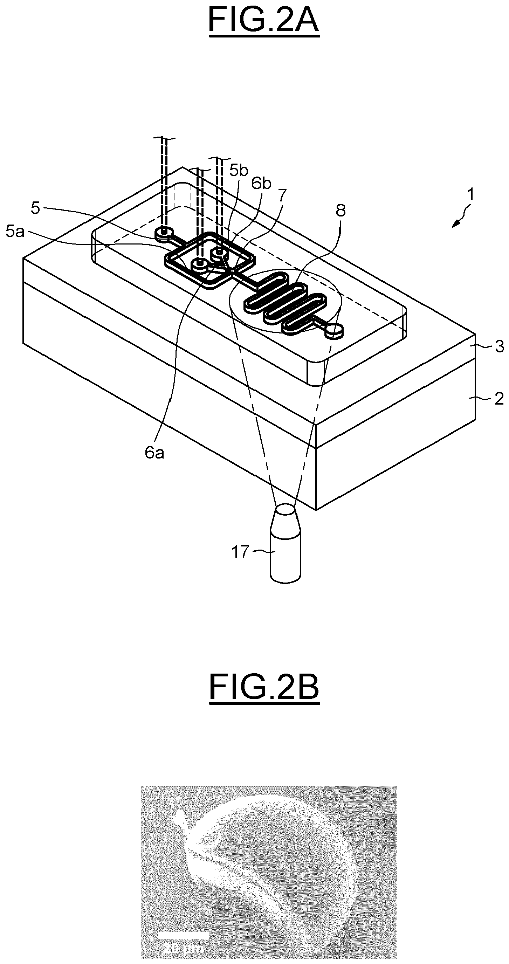

In this embodiment, a microfluidic device is used, as represented in Figure 2A.

As can be seen in Figure 2A, the device 1 may comprise a glass substrate 2, a polydimethylsiloxane layer 3 and a polydimethylsiloxane chip 4, wherein the polydimethylsiloxane layer 3 is located on the glass substrate 2 and the polydimethylsiloxane chip 4 is located on the polydimethylsiloxane layer 3. A channel system (5, 5a, 5b, 18, 18a, 18b, 19, 20, 8) can be found inside the polydimethylsiloxane chip 4.

According to step a), acrylated epoxidised soybean oil can be mixed with at least one solvent, such as for example 1-octanol, and in the presence of at least one photoinitiator, for example phenylbis(2,4,6-trimethylbenzoyl)phosphine oxide (Irgacure^{®} 819).

An aqueous solution comprising at least one surfactant can be prepared. The surfactant can be a non-ionic surfactant, such as for example poloxamer 188 (Pluronic^{®} F68). The aqueous solution can further comprise polyethylene glycol sorbitan monolaurate (Tween 20).

A solution S1 can be prepared, such as a solution S1 consisting of a silicone oil, such as polydimethylsiloxane.

As described in the previous embodiment, the different solutions can be poured into reservoirs and said reservoirs can be connected to a controller, such as for example the one sold under the trade name MFCS-EZ by the company Fluigent.

The whole system (reservoirs, tubes, controller, computer) is not represented in Figure 2A.

The aqueous solution comprising at least one surfactant can be added at a first end of a channel 5 through a tube. The solution obtained after step a) can be added at a first end of a first sub-channel 18a through a tube. The solution S1 can be added at a first end of a second sub-channel 18b through a tube.

The channel 5 can be divided into two sub-channels, a first sub-channel 5a and a second sub-channel 5b.

The first sub-channel 18a and the second sub-channel 18b can join to form a channel 18 at a junction 19.

The solution S1 and the solution obtained after step a) can be contacted at the junction 19 to form a solution obtained after step b) circulating into the channel 18.

Then, the solution obtained after step b) and the aqueous solution comprising at least one surfactant can be contacted thanks to a junction 20 of the first sub-channel 5a, the second sub-channel 5b and the channel 18. Thus, the polydimethylsiloxane chip 4 can incorporate a transverse junction 20 for focusing the flow.

Droplets can thus be formed.

Then, the droplets can follow a coiled third channel 8. This zone can be illuminated using a UV light 17. In other words, the droplets are exposed to a UV light 17. Said UV light 17 can be placed outside the microfluidic device 1 and can be positioned so as to illuminate the coiled third channel 8.

The UV light can have a wavelength ranging from 315 to 380 nm. The exposure to the UV light can be carried out for a period ranging from 0.1 second to 30 minutes, more preferably from 0.5 to 5 seconds, for example for 3 seconds.

At the outlet of the third channel 8 in said chip 4, the acrylated epoxidised soybean oil particles can be retrieved.

Then, said acrylated epoxidised soybean oil particles can be dispersed in a solution comprising at least one photoinitiator, such as for example phenylbis(2,4,6-trimethylbenzoyl)phosphine oxide (Irgacure^{®} 819).

Said solution can further comprise acetone.

Said particles can then be exposed to a UV light. The UV light can have a wavelength ranging from 315 to 380 nm.

The exposure to the UV light can be carried out for a period ranging from 30 seconds to 30 minutes, for example for 2 minutes.

Acrylated epoxidised soybean oil crescent-shaped particles, as represented in Figure 2B, can thus be obtained by the method according to the invention according to a third embodiment.

A fourth embodiment according to the invention can be described hereinafter.

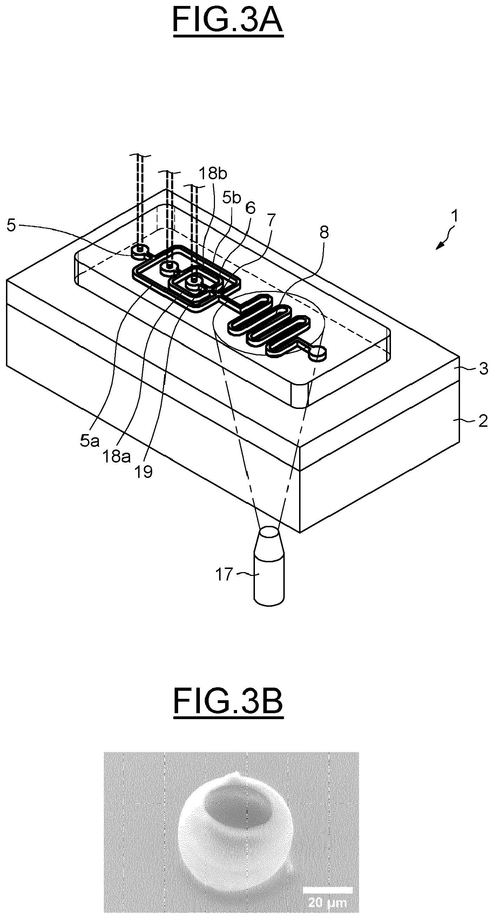

In this embodiment, a microfluidic device is used, as represented in Figure 3A.

As can be seen in Figure 3A, the device 1 may comprise a glass substrate 2, a polydimethylsiloxane layer 3 and a polydimethylsiloxane chip 4, wherein the polydimethylsiloxane layer 3 is located on the glass substrate 2 and the polydimethylsiloxane chip 4 is located on the polydimethylsiloxane layer 3. A channel system (5, 5a, 5b, 21, 21a, 21b, 22, 23, 24, 25, 8) can be found inside the polydimethylsiloxane chip 4.

According to step a), acrylated epoxidised soybean oil can be mixed with at least one solvent, such as for example 1-octanol, and in the presence of at least one photoinitiator, for example phenylbis(2,4,6-trimethylbenzoyl)phosphine oxide (Irgacure^{®} 819).

An aqueous solution comprising at least one surfactant can be prepared. The surfactant can be a non-ionic surfactant, such as for example poloxamer 188 (Pluronic^{®} F68). The aqueous solution can further comprise polyethylene glycol sorbitan monolaurate (Tween 20).

A solution S1 can be prepared, such as a solution S1 consisting of a silicone oil, such as polydimethylsiloxane.

As described in the previous embodiment, the different solutions can be poured into reservoirs and said reservoirs can be connected to a controller, such as for example the one sold under the trade name MFCS-EZ by the company Fluigent.

The whole system (reservoirs, tubes, controller, computer) is not represented in Figure 3A.

The aqueous solution comprising at least one surfactant can be added at a first end of a channel 5 through a tube. The solution obtained after step a) can be added at a first end of a channel 21 through a tube. The solution S1 can be added at a first end of a channel 22 through a tube.

The first channel 5 can be divided into two sub-channels, a first sub-channel 5a and a second sub-channel 5b.

The channel 21 can be divided into two sub-channels, a first sub-channel 21a and a second sub-channel 21b.

The first sub-channel 21a, the second sub-channel 21b and the channel 22 can join to form a channel 24 at a junction 23.

The solution S1 and the solution obtained after step a) can be contacted thanks to the junction 23 to form a solution obtained after step b) circulating into the channel 24.

Then, the solution obtained after step b) and the aqueous solution comprising at least one surfactant can be contacted thanks to a junction 25 of the first sub-channel 5a, the second sub-channel 5b and the channel 24. Thus, the polydimethylsiloxane chip 4 can incorporate two transverse junctions 23 and 25 for focusing the flow.

Droplets can thus be formed.

Then, the droplets can follow a coiled third channel 8. This zone can be illuminated using a UV light 17. In other words, the droplets are exposed to a UV light 17. Said UV light 17 can be placed outside the microfluidic device 1 and can be positioned so as to illuminate the coiled third channel 8.

The UV light can have a wavelength ranging from 315 to 380 nm. The exposure to the UV light can be carried out for a period ranging from 0.1 second to 30 minutes, more preferably from 0.5 to 5 seconds, for example for 3 seconds.

At the outlet of the third channel 8 in said chip 4, the acrylated epoxidised soybean oil particles can be retrieved.

Then, said acrylated epoxidised soybean oil particles can be dispersed in a solution comprising at least one photoinitiator, such as for example phenylbis(2,4,6-trimethylbenzoyl)phosphine oxide (Irgacure^{®} 819).

Said solution can further comprise acetone.

Said particles can then be exposed to a UV light. The UV light can have a wavelength ranging from 315 to 380 nm.

The exposure to the UV light can be carried out for a period ranging from 30 seconds to 30 minutes, for example for 2 minutes.

Acrylated epoxidised soybean oil particles having a hole-shell structure, as represented in Figure 3B, can thus be obtained by the method according to the invention according to a fourth embodiment.

A fifth embodiment according to the invention can be described hereinafter.

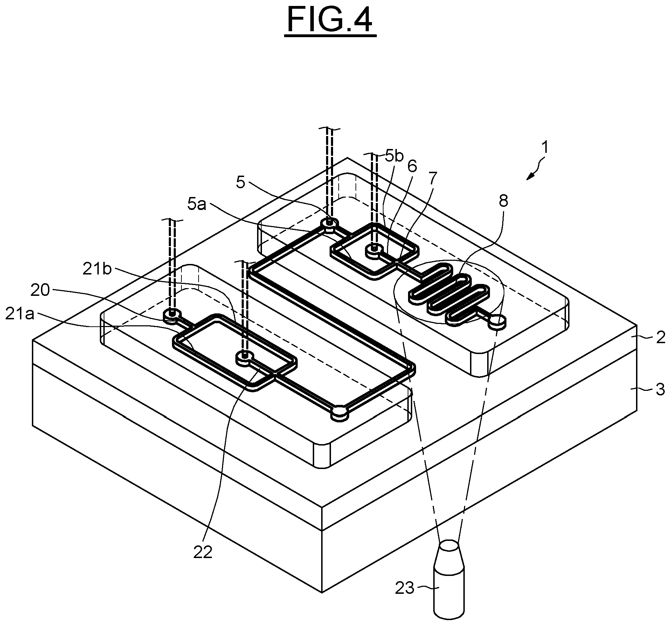

In this embodiment, a microfluidic device is used, as represented in Figure 4.

As can be seen in Figure 4, the device 1 may comprise a glass substrate 2, a polydimethylsiloxane layer 3, a first polydimethylsiloxane chip 4a and a second polydimethylsiloxane 4b connected in series, wherein the polydimethylsiloxane layer 3 is located on the glass substrate 2 and the first and second polydimethylsiloxane chip 4a, 4b are located on the polydimethylsiloxane layer 3. A channel system (26, 26a, 26b, 27, 28, 29, 30, 31, 31a, 31b, 32, 33, 8) can be found inside the polydimethylsiloxane chips 4a and 4b, as can be seen in Figure 4.

According to step a), acrylated epoxidised soybean oil can be mixed with at least one solvent, such as for example 1-octanol or methyl oleate, and in the presence of at least one photoinitiator, for example phenylbis(2,4,6-trimethylbenzoyl)phosphine oxide (Irgacure^{®} 819), and of an optional surfactant, such as span 80.

An aqueous solution comprising at least one surfactant can be prepared. The surfactant can be a non-ionic surfactant, such as for example poloxamer 188 (Pluronic^{®} F68). The aqueous solution can further comprise polyethylene glycol sorbitan monolaurate (Tween 20).

A solution S1 can be prepared, such as an aqueous solution S1 comprising a viscosity agent, such as alginate sodium.

As described in the previous embodiment, the different solutions can be poured into reservoirs and said reservoirs can be connected to a controller, such as for example the one sold under the trade name MFCS-EZ by the company Fluigent.

The whole system (reservoirs, tubes, controller, computer) is not represented in Figure 4.

The solution obtained after step a) can be added at a first end of a channel 26 through a tube. The solution S1 can be added at a first end of a channel 27 through a tube.

The channel 26 can be divided into two sub-channels, a first sub-channel 26a and a second sub-channel 26b.

The first sub-channel 26a, the second sub-channel 26b and the channel 27 can join to form a channel 29 at a junction 28.

The solution S1 and the solution obtained after step a) can be contacted thanks to the junction 28 to form a solution obtained after step b) circulating into the channel 29.

Water-in-oil droplets can thus be formed.

The aqueous solution comprising at least one surfactant can be added at a first end of a channel 31 through a tube.

The channel 29 is connected to a first end of a channel 32 through a channel 30.

The channel 31 can be divided into two sub-channels, a first sub-channel 31a and a second sub-channel 31b.

Then, the solution obtained after step b) and the aqueous solution comprising at least one surfactant can be contacted thanks to a junction 33 of the first sub-channel 31a, the second sub-channel 31b and the channel 32. Thus, the polydimethylsiloxane chip 4 can incorporate a transverse junction 33 for focusing the flow.

Water-in-oil-in-water droplets can thus be formed.

Then, the droplets can follow a coiled third channel 8. This zone can be illuminated using a UV light 17. In other words, the droplets are exposed to a UV light 17. Said UV light 17 can be placed outside the microfluidic device 1 and can be positioned so as to illuminate the coiled third channel 8.

The UV light can have a wavelength ranging from 315 to 380 nm. The exposure to the UV light can be carried out for a period ranging from 0.1 second to 30 minutes, more preferably from 0.5 to 5 seconds, for example for 3 seconds.

At the outlet of the third channel 8 in said chip 4b, the acrylated epoxidised soybean oil particles can be retrieved.

Then, said acrylated epoxidised soybean oil particles can be dispersed in a solution comprising at least one photoinitiator, such as for example phenylbis(2,4,6-trimethylbenzoyl)phosphine oxide (Irgacure^{®} 819).

Said solution can further comprise acetone.

Said particles can then be exposed to a UV light. The UV light can have a wavelength ranging from 315 to 380 nm.

The exposure to the UV light can be carried out for a period ranging from 30 seconds to 30 minutes, for example for 2 minutes.

Acrylated epoxidised soybean oil microcapsules particles can thus be obtained by the method according to the invention according to a fifth embodiment. Sais microcapsules can have shell and core parts.

The invention also relates to acrylated epoxidised plant oil particles obtained by the method according to the invention.

Preferably, the acrylated epoxidised plant oil particles are epoxidised acrylated soybean oil particles.

The invention also relates to the use of acrylated epoxidised plant oil particles obtained by the method according to the invention for charging fluorescent molecules or for releasing biological molecules, such as ibuprofen, or as optical lens.

The present invention is illustrated in a non-limiting manner by the following examples.

### Examples

### Example 1: method for preparing acrylated epoxidised soybean oil particles

Acrylated epoxidised soybean oil is added to 1-octanol. phenylbis(2,4,6-trimethylbenzoyl)phosphine oxide (Irgacure^{®} 819) is also added. The acrylated epoxidised soybean oil content is 85% by weight with respect to the total weight of the solution. The phenylbis(2,4,6-trimethylbenzoyl)phosphine oxide (Irgacure^{®} 819) content is 0.4% by weight with respect to the total weight of the solution.

Said solution obtained is mixed with an aqueous solution comprising poloxamer 188 (Pluronic^{®} F68) and sodium alginate. The poloxamer 188 (Pluronic^{®} F68) content is 15% by weight with respect to the total weight of said aqueous solution. The sodium alginate content is 2% by weight with respect to the total weight of said aqueous solution.

Both solutions are mixed in a vortex mixer.

The droplets obtained are then retrieved, isolated, and then rinsed with a solution comprising polyethylene glycol sorbitan monolaurate (Tween 20; 0.06 mmol/L, critical micelle concentration (CMC)) and a phosphate buffer (20 mmol/L, pH about of 7). This rinsing of the droplets makes it possible to remove the surfactant and the viscosity agent.

The droplets are then exposed to a UV light, of a wavelength ranging from 315 to 380 nm and having a power of 60 mW/cm². The exposure to UV light is carried out for 2 minutes.

The acrylated epoxidised soybean oil particles thus obtained are of spherical shape and have a smooth appearance. They have an average diameter (average Feret diameter) of 34.7 µm ± 10.1 µm.

### Example 2: method for preparing acrylated epoxidised soybean oil particles

Acrylated epoxidised soybean oil is added to 1-octanol. phenylbis(2,4,6-trimethylbenzoyl)phosphine oxide (Irgacure^{®} 819) is also added. The acrylated epoxidised soybean oil content is 65% by weight with respect to the total weight of the solution. The phenylbis(2,4,6-trimethylbenzoyl)phosphine oxide (Irgacure^{®} 819) content is 0.4% by weight with respect to the total weight of the solution.

Said solution obtained is mixed with an aqueous solution comprising poloxamer 188 (Pluronic^{®} F68). The poloxamer 188 (Pluronic^{®} F68) content is 15% by weight with respect to the total weight of said aqueous solution.

In this example, a microfluidic device device is used, as represented in Figures 1A and 1B.

As can be seen in Figure 1A, the device 1 comprises a glass substrate 2, a polydimethylsiloxane layer 3 and a polydimethylsiloxane chip 4, wherein the polydimethylsiloxane layer 3 is located on the glass substrate 2 and the polydimethylsiloxane chip 4 is located on the polydimethylsiloxane layer 3. A channel system (5, 5a, 5b, 6, 7, 8) is found inside the polydimethylsiloxane chip, as can be seen in Figure 1B.

The aqueous solution is then poured into a reservoir 9 and said reservoir 9 is connected to a first end of a channel 5 through a tube 10. The solution containing acrylated epoxidised soybean oil is poured into a reservoir 11 and said reservoir 11 is connected to a first end of a channel 6 through a tube 12.

The reservoir 9 is connected to a controller 13 (sold under the trade name MFCS-EZ by the company Fluigent), the controller 13 being connected to a computer 14, through a tube 15. The reservoir 11 is connected to the controller 13 through a tube 16. Thanks to the controller 13, air is pumped through tubes 15 and 16, respectively.

The solution containing acrylated epoxidised soybean oil is added with a pressure of 133 mbar at a first end of a channel 5 and the aqueous solution is added with a pressure of 138 mbar at a first end of a channel 6, as represented in Figure 1B. The channel 5 is divided into two sub-channels, a first sub-channel 5a and a second sub-channel 5b.

Then, both solutions are contacted thanks to a junction 7 of the first sub-channel 5a, the second sub-channel 5b and the channel 6. Thus, the polydimethylsiloxane chip 4 incorporates a transverse junction 7 for focusing the flow.

Droplets are thus formed.

Then, the droplets follow a coiled third channel 8, as can be seen in Figure 1B. This zone is illuminated using a UV light 17. Thus, the droplets are exposed to a UV light 17. Said UV light 17 is placed outside the microfluidic device 1 and is positioned so as to illuminate the coiled third channel 8.

The UV light has a wavelength ranging from 315 to 380 nm and has a power of 424 mW/cm². The exposure to UV light is carried out for 1 second.

At the outlet of the third channel 8 in said chip 4, the acrylated epoxidised soybean oil particles are retrieved and collected in a microtube, then centrifuged with 3000 rad/min for 30 s and rinsed three times with acetone to remove poloxamer 188 (Pluronic^{®} F68) and 1-octanol in excess.

Then, said acrylated epoxidised soybean oil particles are dispersed in a solution comprising 0,4% by weight of phenylbis(2,4,6-trimethylbenzoyl)phosphine oxide (Irgacure^{®} 819) and acetone.

Said particles are then exposed to a UV light. The UV light has a wavelength ranging from 315 to 380 nm and has a power of 60 mW/cm². The exposure to UV light is carried out for 2 minutes.

Acrylated epoxidised soybean oil particles can thus be obtained with the method according to the invention.

The particles thus obtained have a very narrow size distribution with an average diameter (average Feret diameter) of 46.6 µm ± 1.2 µm.

### Example 3: fluorescent molecules

Specific modified fluorescent phospholipids (DOPE-CF (1,2-dioleoyl-sn-glycero-3-phosphoethanolamine-N-(carboxyfluorescein), sold by Avanti Polar Lipids (Alabaster, AL, USA)) were added to the solution as prepared during step a), according to a concentration of 0.1 mg/mL.

Then, the protocol followed is in accordance with that implemented in example 2 above. Thus, the manufacturing method involves the use of the microfluidic device as described in example 2.

The particles obtained were then excited by a 488nm laser.

It was observed that the particles obtained had a homogeneous green fluorescence which can be maintained during the exposure to UV.

### Example 4: degradability of the particles

The acrylated epoxidised soybean oil particles as manufactured in example 2 were contacted in different solutions A, B, C, D and E.

Solution A is an aqueous solution containing 3% by weight of potassium hydroxide at ambient temperature.

Solution B is an ethanolic solution containing 3% by weight of potassium hydroxide at ambient temperature.

Solution C is an ethanolic solution containing 3% by weight of potassium hydroxide and heated to 70°C.

Solution D is an aqueous solution containing 150 units/mL of esterase solution at ambient temperature.

Solution E is an aqueous solution containing 700 units/mL of lipase at ambient temperature.

Said particles exhibit the highest degradation rate in solution C. Indeed, the particle suspension becomes transparent and the particles are completely hydrolysed. After 1 hour of incubation, no particle is observed under the microscope.

On the other hand, 19 hours were needed to observe complete particle degradation with solution B.

Moreover, even after 277 hours of incubation in solution A, particle fragments were still observed.

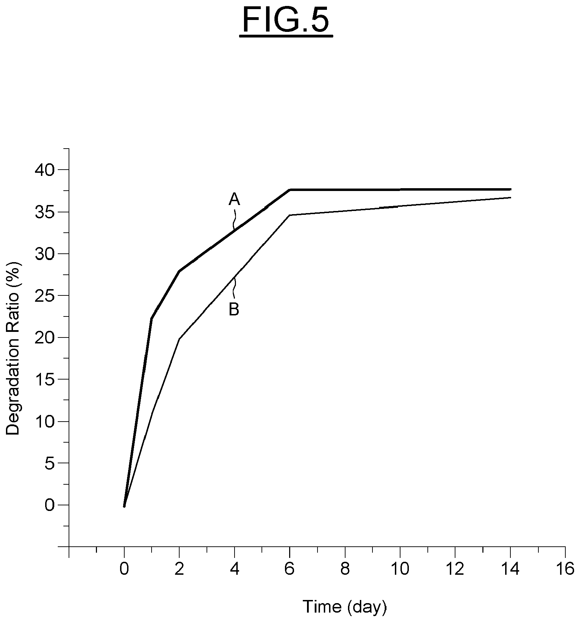

It was observed that the particles had a good enzymatic degradability in both solutions D (curve B) and E (curve A) in a short time, as can be seen in Figure 5.

The particles have a more rapid hydrolysis rate under the effect of lipase in the initial phase (curve A). Then, the hydrolysis rates of the particles in both solutions gradually slow down and plateau after 6 days, at which time about 35% of the particles have been degraded (curves A and B).

Thus, the particles obtained with the method according to the invention can be degraded in aqueous phase in the presence of enzymes or in an alcoholic and alkaline solution.

This is a very advantageous property compared to the materials used extensively in various fields at the present time which are not degradable and/or nonrecyclable.

### Example 5: releasing of ibuprofen

A similar method to the one used in example 1 was used here. More specifically, 15mg Ibuprofen ((RS)-2-(4-(2-Methylpropyl)phenyl)propanoic acid) was added into 1mL AESO/1-Octanol solution, which contained 85% by weight AESO and 0.4% by weight Irgacure 819 photoinitiator. Ibuprofen-loaded AESO particles were obtained.

Then, said particles were contacted in two different environments: simulated intestinal fluid [SIF, 6.8 g KH₂PO₄ and 77 mL aqueous NaOH (0.2 M) in water - final pH = 6.8, 1000 mL] and simulated gastric fluid [SGF, 2.0 g NaCl and 7.0 mL HCl (37%) in water - final pH = 1.2, 1000 mL], both without the presence of enzymes.

The release tests were carried out through incubating ibuprofen-loaded AESO particles in biomimetic environments, in which for each experiment 50 mg of dry loaded particles were deposited in a sealed flask with 50 ml of SIF or SGF.

After the immersing of each sample in SIF and SGF, respectively, the system was kept under constant shaking of 140 rpm at 37°C over the whole test. At different time interval (0.5, 1, 2, 4, 8, 10, 24, 48, 120, 144 h), an aliquot (70 µL) was taken from each flask to quantify, through UV-Vis spectrum measurements, the amount of drug released. The aliquots were centrifuged before the UV-Vis analysis to avoid the interference of particles.

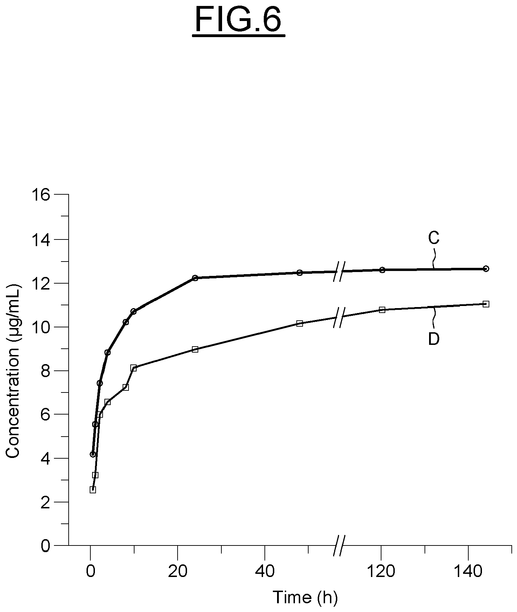

The evolution of the concentration of ibuprofen in the simulated intestinal fluid as a function of time (curve C) and the evolution of the concentration of ibuprofen in the simulated gastric fluid as a function of time (curve D) were observed, as can be seen in Figure 6.

It can be seen that the ibuprofen-loaded AESO particles possessed a more rapid releasing rate in the simulated intestinal fluid than in the simulated gastric fluid.

The releasing rates of the particles in both environments gradually slowed down and reached a plateau after 120 h, at which time the concentration of ibuprofen in the solution is 12.5 µg/mL (SIF) and 10.9 µg/mL (SGF), which corresponded to a drug release rate of 83.7% and 73%, respectively.

## Claims

1. Method for manufacturing acrylated epoxidised plant oil particles comprising the following steps:
a) mixing acrylated epoxidised plant oil with at least one solvent and in the presence of at least one photoinitiator;
b) optionally mixing the solution after step a) with a solution S1;
c) mixing the solution obtained after step a) or after optional step b) with an aqueous solution comprising at least one surfactant in order to obtain droplets;
d) exposing said droplets to a UV light;
e) retrieving the acrylated epoxidised plant oil particles obtained after step d).

2. Method according to claim 1, **characterised in that** the acrylated epoxidised plant oil is chosen from acrylated epoxidized linseed oil, acrylated castor oil, acrylated epoxidised soybean oil and their mixtures, preferably the acrylated epoxidised plant oil is acrylated epoxidised soybean oil.

3. Method according to claim 1 or 2, **characterized in that** the acrylated epoxidised plant oil particles are present at a content ranging from 40 to 90% by weight, preferably from 50 to 90% by weight, more preferably from 60 to 90% by weight, even more preferably from 65 to 85% by weight with respect to the total weight of the solution obtained after step a).

4. Method according to any one of the preceding claims, **characterized in that** the solvent is chosen from alcohols, esters and their mixtures, preferably from C₆ to C₁₂ monoalcohols, C₁₂ to C₃₆ esters and their mixtures, more preferably from C₆ to C₁₂ monoalcohols, C₁₂ to C₂₄ esters and their mixtures, even more preferably from 1-octanol, methyl oleate and their mixture, even better the solvent is 1-octanol.

5. Method according to any one of the preceding claims, **characterized in that** the photoinitiator is chosen from ketones, phosphine oxides and their mixtures, preferably from 2-hydroxy-4'-(2-hydroxyethoxy)-2-methylpropiophenone, phenylbis(2,4,6-trimethylbenzoyl)phosphine oxide and their mixture, even more preferably the photoinitiator is phenylbis(2,4,6-trimethylbenzoyl)phosphine oxide.

6. Method according to any one of the preceding claims, **characterized in that** the photoinitiator is present at a content ranging from 0.05 to 5% by weight, preferably from 0.05 to 3% by weight, more preferably from 0.05 to 1% by weight, even more preferably from 0.1 to 0.5% by weight with respect to the total weight of the solution obtained after step a).

7. Method according to any one of the preceding claims, **characterized in that** the solution S1 is chosen from a solution comprising water and a solution comprising a silicone oil.

8. Method according to any one of the preceding claims, **characterized in that** the surfactant is chosen from anionic surfactants, non-ionic surfactants, and their mixtures, preferably from non-ionic surfactants, more preferably from poloxamer 188.

9. Method according to any one of the preceding claims, **characterised in that** step d) is carried out for a period ranging from 0.1 second to 30 minutes, preferably from 0.5 second to 20 minutes, more preferably from 0.5 second to 10 minutes.

10. Method according to any one of the preceding claims, **characterised in that** a step of isolating and rinsing the droplets is carried out between step c) and step d).

11. Method according to any one of claims 1 to 9, **characterised in that** optional step b), and steps c) and d) are carried out in a microfluidic device comprising a glass substrate, a polydimethylsiloxane layer and a polydimethylsiloxane chip, and wherein the polydimethylsiloxane layer is located on the glass substrate and the polydimethylsiloxane chip is located on the polydimethylsiloxane layer.

12. Method according to claim 11, **characterised in that** the method comprises a step f) of dispersing said acrylated epoxidised plant oil particles obtained after step e) in a solution comprising at least one photoinitiator, followed by exposure to a UV light.

13. Method according to claim 12, **characterised in that** the exposure to UV light during step f) is carried out for a period ranging from 30 seconds to 30 minutes, preferably from 1 minute to 10 minutes, more preferably from 1 minute to 5 minutes.

14. Acrylated epoxidised plant oil particles obtained by the method as defined in any one of the preceding claims.

15. Use of the acrylated epoxidised plant oil particles as defined in claim 14 or as obtained by the method as defined in any one of claims 1 to 13 for charging fluorescent molecules or for releasing biological molecules, such as ibuprofen, or as optical lens.
